(19) 

Europäisches
Patentamt
European
Patent Office
Office européen
des brevets

(11) **EP 1 562 480 B1**

(12) **EUROPEAN PATENT SPECIFICATION**

(45) Date of publication and mention
of the grant of the patent:
**22.10.2008 Bulletin 2008/43**

(51) Int Cl.:
*A61B 8/00* *(2006.01)*   *G01K 11/26* *(2006.01)*
*A61N 7/02* *(2006.01)*

(21) Application number: **03773015.7**

(86) International application number:
**PCT/SE2003/001783**

(22) Date of filing: **18.11.2003**

(87) International publication number:
**WO 2004/045413 (03.06.2004 Gazette 2004/23)**

(54) **METHOD AND APPARATUS FOR NON-INVASIVE MEASUREMENT OF A TEMPERATURE CHANGE INSIDE A LIVING BODY**

VERFAHREN UND GERÄT FÜR DIE NICHTINVASIVE MESSUNG EINER TEMPERATURVERÄNDERUNG IN EINEM LEBENDEN KÖRPER

PROCEDE ET APPAREIL DE MESURE NON INVASIVE D'UNE VARIATION DE TEMPERATURE DANS UN CORPS VIVANT

(84) Designated Contracting States:
**AT BE BG CH CY CZ DE DK EE ES FI FR GB GR HU IE IT LI LU MC NL PT RO SE SI SK TR**

(30) Priority: **18.11.2002 SE 0203396**

(43) Date of publication of application:
**17.08.2005 Bulletin 2005/33**

(73) Proprietor: **Sunnanväder, Lars
72379 Hechingen (DE)**

(72) Inventor: **Sunnanväder, Lars
72379 Hechingen (DE)**

(74) Representative: **Karlsson, Leif Gunnar Börje et al
Ström & Gulliksson AB
P.O. Box 4188
203 13 Malmö (SE)**

(56) References cited:
**US-A- 4 807 633**

- **PATENT ABSTRACTS OF JAPAN vol. 013, no. 557 (P-974), 12 December 1989 (1989-12-12) & JP 01 233337 A (NIPPON TELEGR & TELEPH CORP), 19 September 1989 (1989-09-19)**
- **Seip R. et al.: "Invasive and Non-invasive Feedback for Ultrasound Phased Array Thermometry", 1994 IEEE Ultrasonics Symposium, pages 1821-1824**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 1 562 480 B1**

**Description**

***Background of the invention***

*Field of the invention*

The invention relates generally to method and apparatus for non-invasive measurement of a change of the temperature inside a living body, from the outside or a cavity of the living body.

When an ultrasound wave passes through a material such as body tissue energy of the ultrasonic wave is absorbed and the wave is attenuated. The part of the ultrasonic wave that is absorbed is converted substantially to heat energy. Therapeutic ultrasound i.-e. high intense ultrasound is used for heat treatment of i. a. muscles and other structures in the living body mostly in order to increase the blood flow through the structure and to speed up the healing thereof. The heating by therapeutic ultrasound is effected up to about 3 W/cm$^2$. The temperature of the heated structure and the surrounding tissue is not controlled. Recently the MR-technique has been used, but it is an expensive a slow technique. Therefore, in this case as when tissue is destructed by local heating to a temperature of 42 to 95° or when a stent inserted into a blood vessel is heated for heat treatment of abnormalities in the vessel reliable control of the temperature change in the tissue or the stent during the treatment is desired in order to effect the treatment without injuring tissue surrounding the region to be treated by ultrasound heating.

*Description of the Prior Art*

The article by Seip R. et al.; "Invasive and Non-invasive Feedback for Ultrasound Phased Array Thermometry", 1994 IEEE Ultrasonics Symposium, pages 1821-1824, discloses a method and apparatus for measuring the temperature change of a target inside by body according to the preamble of, respectively, claims 1 and 8.

It is also known in the art to provide temperature control during non-invasive treatment of a target area inside a living body. Thus, JP-A-1233337 describes method and apparatus for measuring temperature of an object which cannot be approached from the outside, such as tissue inside a human body, which is heated by external heater, wherein an ultrasonic wave is transmitted to the object. A reflected wave therefrom is subjected to A/D conversion and the sensed wave is cut out at each one wavelength for frequency analysis. The temperature of the object is calculated therefrom on the basis of a predetermined relationship between a peak frequency of a frequency distribution obtained by the analysis, or a half-width thereof, and the temperature of the object.

US-A-5 370 121 describes method and apparatus for non-invasively measuring a temperature change between two points in a region of interest in the inside of a living subject which is treated with heating radiation. A first ultrasound wave form, containing at least one ultrasound pulse is emitted into the subject at a first point of time and is incident on the region of interest which reflects a corresponding first set of echo signals from which a first ultrasound image is generated and stored. A second ultrasound waveform identical to the first ultrasound wave form is emitted into the subject at a second point in time incident on said region which reflects a corresponding second set of echo signals. A second ultrasonic image is generated therefrom. From the first and second ultrasonic images a differential ultrasound image is generated. A temperature change, if any, in the region of interest is identified during the course of producing ultrasound images of the region by determining the change of the acoustic impedance of the region of interest between the first and second points in time, a temperature change being allocated to the impedance change.

US-A-4 807 633 describes method and apparatus for non-invasive thermometry which is based on the observation that attenuation of a transmitted or reflected beam of ultrasound in tissue changes measurably as tissue temperature changes. The prior art method and apparatus comprise periodic amplitude measurement of back-scattered ultrasound.

US-A-6 050 943 relates to a combined imaging , therapy and temperature monitoring system comprising an acoustic transducer assembly configured to enable the ultrasonic system to perform the imaging, therapy and temperature monitoring functions. The transducer assembly includes a single transducer which is operatively connected to an imaging subsystem, a therapy subsystem and a temperature monitoring subsystem. The therapy subsystem can generate high power acoustic energy to heat a treatment region, and the temperature monitoring subsystem can map and monitor the temperature of tissue in said region and display the temperature on a display. Mapping of the temperature distribution in tissue in the treatment region is accomplished by measuring the time-of-flight and amplitude data of acoustic pulses through said region while exploiting the temperature dependence of the speed of sound and acoustic attenuation in the tissue of the treatment region. Imaging, temperature heating and temperature monitoring of the treatment region can be conducted substantially simultaneously.

US 4 566 460 describes method and apparatus for measuring a non-linear parameter A/B of an acoustic medium or its distribution, and the application of the parameter to non-invasive measurement of internal temperature of a sample. A continuous wave ultrasonic probing beam is radiated through the sample, and a pumping wave which is an ultrasonic pulse is superposed on the probing beam. A phase change in the probing beam caused by the pumping wave is detected and from this phase change the non-linear parameter (B/A) is obtained. From the information concerning the variation of the measured value of said parameter the inner temperature of the sample is obtained.

US 5 360 268 describes an ultrasonic temperature measuring apparatus measuring the temperature of a medium, which includes a transmitter for transmitting ultrasonic waves and a receiver for receiving the ultrasonic waves and providing a received signal. An operation unit calculates the propagation time of the ultrasonic waves, and the temperature in the medium is determined according to the propagation time of the ultrasonic waves.

US 4 817 615 describes an ultrasonic diagnostic apparatus which includes an ultrasonic transducer for transmitting an ultrasonic wave into a body to be examined and receiving the reflected ultrasonic wave, phase variations being detected to obtain fluctuation of the temperature within the body.

A similar ultrasonic pulse temperature determination method and apparatus are described in US 4 754 760 wherein ultrasound pulses are transmitted into a specimen and the attenuation of the ultrasound pulses between different depths in the specimen is determined before and after heating of the specimen. The change in temperature of the specimen is determined from the obtained attenuation values.

JP 61280533 describes an apparatus for measuring internal temperature of a living body by reflected ultrasonic wave delivered to the interior of the living body. Spectral analysis is applied to the frequency of the reflected wave and the change quantity of the frequency spectrum of the reflected wave is calculated and is converted to a temperature change quantity.

The prior art methods and apparatuses do not provide the precision in measuring the temperature of the target as is required in order to provide an accurate temperature control for an adequate therapeutic treatment, and/or require use of several measuring points, which makes the use of the prior art methods and apparatuses rather complicated.

### Brier Summary of the Invention

The object of the invention is to overcome the drawbacks mentioned above and to provide method and apparatus for accurate measuring of the temperature in a very small area of a target within a region which is ultrasonically heated, especially for treating a vessel using stent material or for destructing tissue such as cancer tissue, by using back scattered ultrasound from the target for the temperature measurements.

More particularly the invention provides method and apparatus allowing control of the temperature in a target heated by pulsed therapeutic high intense ultrasound, after each therapeutic pulse in order to produce a well adjusted energy dose for creating an adequate temperature of a confined small area of the target.

In order to achieve the above object the invention provides a method according to claim 1 and an apparatus according to claim 8.

Further features of the invention are defined in the dependent claims.

### Brief Description of the Drawing

FIG 1 is a block diagram of an apparatus of the invention,
FIG 2 is a time diagram of the procedure applied when using the apparatus of FIG 1,
FIG 3 is a frequency spectrum of a pulse of back scattered ultrasound, and
FIGS 4 and 5 are diagrams illustrating the frequency peaks of the harmonics of the back scattered pulse.

### Detailed Description of the Invention

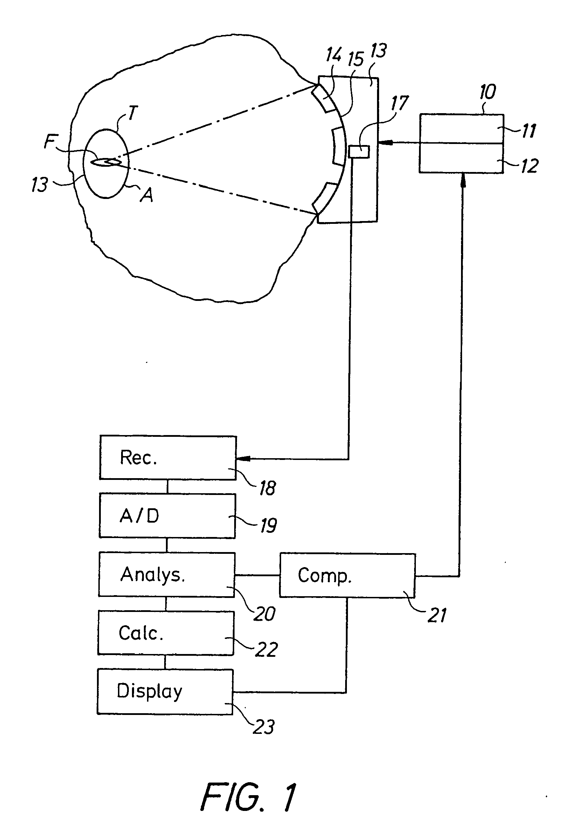

Referring to FIG 1 the apparatus for practising the method of the invention comprises a control unit 10 including a transmitter 11 for generating low intense (diagnostic) ultrasound energy for temperature measuring, and a transmitter 12 for generating high intense therapeutic ultrasound energy, the ultrasound energy being transmitted by a transducer 13 comprising a number of ultrasound emitters formed by thin ceramic plates 14 mounted to a reflecting bowl shaped mounting system 15 focusing the transmitted ultrasound energy. The two transmitter circuits can also be combined to form a single circuit for therapeutic as well as diagnostic purposes. Therapeutic ultrasound energy generated by the transmitter 12 is emitted from the transducer 13 which is applied against an outside surface of the body tissue, for treatment of the tissue in a target region T of body tissue e. g. cancer tissue, to be treated, or to a stent located in a blood vessel. It is also possible to use phased array transducer systems both for treating and temperature measuring instead of the described bowl shaped transducer arrangement. The target region T is located between first and second tissue surfaces A and B. The thickness of the target region T between surfaces A and B is defined by applying one or the other of prior art methods developed for said purpose. Transducer 13 is adjusted in focus the emitted ultrasound energy on an area F located in the target region T. In FIG 1 the focused area F in the target region T has an ellipsoid form of a size which is substantially the same as that of a grain of rice but can be smaller or larger depending on the construction. A sensor 17 is provided in transducer 13 for picking up back scattered ultrasound echo signals. A receiver 18 including a wide band amplifier with controlled amplification is provided for receiving and amplifying the picked up ultrasound echo signals. Receiver 18 is connected to an analogue/digital converter 19 with memory and with a high sampling frequency fs ranging from > 3 x fo to about 20 x fo where fo is the fundamental frequency (first harmonic) of the echo signal, for converting signals received by the receiver from analogue form to digital form in order to facilitate subsequent signal processing. fo can be of different frequency with a variation in bandwidth for optimal temperature sensitivity. Output signals from the receiver are transmitted via the converter to an analyser 20 which can be an FFT (fast Fourier transform) analyser or a Doppler analyser or wavelet technique or an analyser correlating echoes from different types and configuration of transmitted ultrasound pulses. A single analyser of one of the types mentioned or a combination thereof can be provided. The output signal from the analyser (or each analyser) is transferred to a complex comparing circuit 21 herein referred to as a comparator wherein the signal is compared with a reference stored therein. As an optional (used or not used) feature comparator 21 is operatively connected to transmitter 10. When a comparison indicates that the input signal equals a pre-set reference value the comparator shuts of transmitter 12. Analyser 20 is connected to a calculator 22 including programs for processing the received back scattered echo signals. A display 23 is connected to comparator 21 and a calculator 22 for visually presenting the parameters of interest.

Parameters of the treatment such as ultrasound intensity, type of pulse sequence, depth of target region T, frequency of the ultrasound, and selected temperature are programmed or set on control unit 10 and measure the temperature in every focal point.

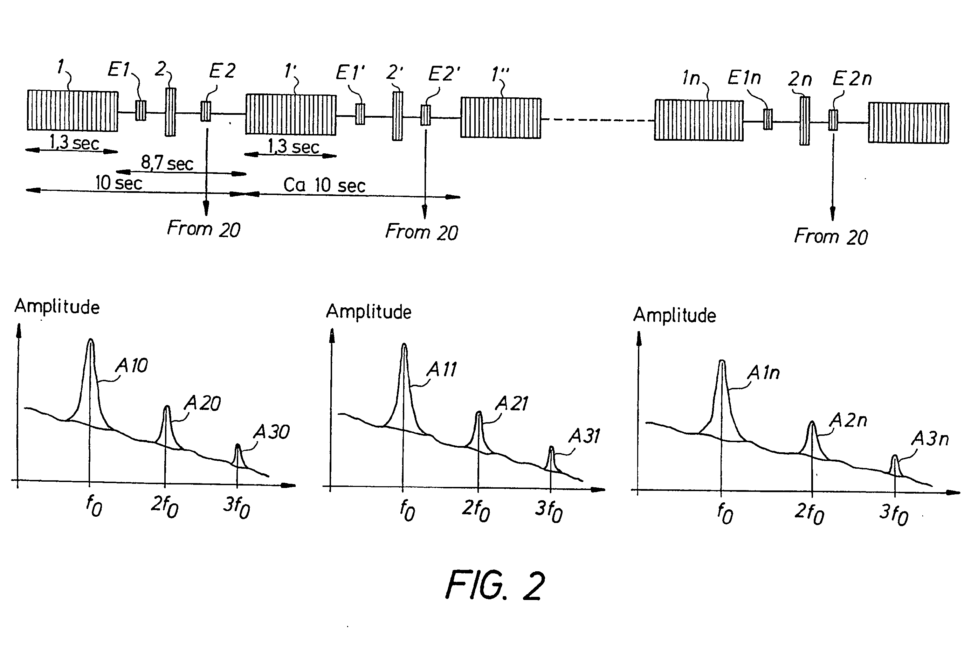

With reference to FIG 2 which illustrates diagrammatically a typical sequence for effecting a non-invasive treatment by means of therapeutic high intense ultrasound the several steps being marked on a time axis.

A therapeutic ultrasound pulse 1 is emitted from the apparatus for about 1.3 seconds and then there is a pause to the next therapeutic ultrasound pulse 1' for a period of 8.7 seconds. This can also be scaled down by approximately a factor of 10, and also the pulse duration quote can be changed. During the pause of 8.7 seconds between pulses 1 and 1' a temperature diagnostic pulse 2 from transmitter 11 is emitted by transducer 13 and the result of the treatment is checked by using the back scattered echo E2 from pulse 2 and the back scattered echo E2' from pulse 2' or, generally, by using the back scattered echo E2n from pulse 2n.

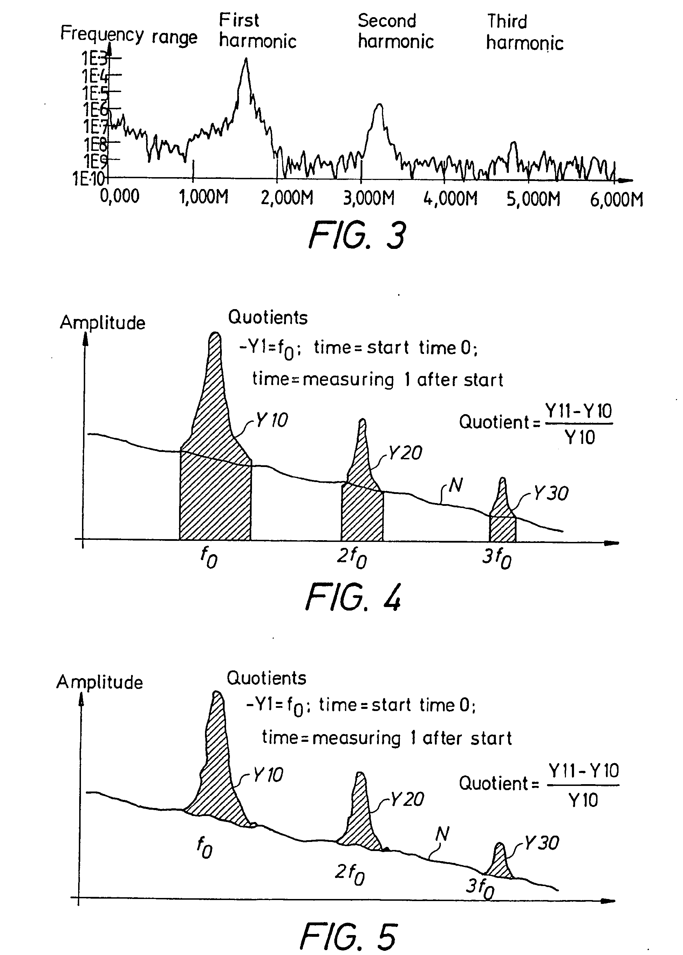

The signal representing the back scattered echo from the target region T under treatment by means of the therapeutic high intense ultrasound is presented as a frequency spectrum as disclosed in FIG 3 by analyser 20. This spectrum comprises a first harmonic (fundamental frequency) A1, a second harmonic A2, a third harmonic and possibly further high order harmonics. The first, second and third harmonics have the frequency f0, 2f0 and 3f0, respectively. The amplitudes of these frequencies is represented by analyser 20 as shown in FIG. 2.

Measurement of echo E2 is the first measurement and is designated 0, and the amplitude of three harmonics included in said echo are designated A10 at the frequency f0, A20 at the frequency 2f0, and A30 at the frequency 3f0.

The measurement of echo E2' is the second measurement and is designated 1. The corresponding three harmonics are designated A11, A21 and A31.

At measurement n of an echo caused by a diagnostic temperature measurement pulse 2n the three harmonics are designated A1n, A2n and A3n.

The echo of the tracking end of the therapeutic pulse 1, 1' and 1n are indicated in FIG. 2 at E1, E1' and E/n and can be used as measurement pulses but it is preferred to use separate pulses 2, 2' and 2n for this purpose. Referring to FIG. 2 the first simplest quotient is calculated for each measuring point at E1 and E2 and for the E1n and E2n by the formula:

$$\text{quotient} = (\text{Amplitude of second harmonic/Amplitude of first harmonic})$$

or

$$\text{quotient} = (\text{Intensity integral of second harmonic/Intensity integral of first harmonic})$$

as illustrated in FIG. 3.

By means of a program in the calculator 22 the quotient,

$$\frac{A1n - A10}{A10}$$

is calculated, wherein A10 is the amplitude of the first harmonic before start of treatment and A1n is the amplitude of the first harmonic at measurement n after start.

An other quotient,

$$\frac{A2n - A20}{A20}$$

is calculated, wherein A20 is the amplitude of the second harmonic before start of treatment and A20 is the amplitude of the second harmonic at measurement and A2n is the amplitude of the second harmonic at measurement n after start.

Calculations can be made for following measurements the measurement to provide the quotient for the second harmonic and the quotient between second harmonic and first harmonic

$$\frac{A2n - A2(n-1)}{A2(n-1)}$$

were n-1 is the measurement just before measurement nr n. The invention is based on the findings that the amplitude quotient of harmonics (as well as intensity quotient, based on the square of the amplitude) is dependent of the temperature change in the target region during heating thereof. Thus it has been found that there is an almost linear relationship between the temperature change and the quotient

$$\Delta T = C \times the\ quotient$$

wherein C is a constant factor that is determined empirically and is specific for tissue type, depth of target region T, ultrasound, intensity and frequency and the sensor system applied .

With reference to FIG 4 as an alternative to the amplitude quotient an integral quotient can be used the area of the peaks in the diagram down to the horizontal axis for the amplitude zero being used for the calculation of the quotient

$$\frac{Y21 - Y20}{Y20}$$

or as shown in Fig 5 the quotient is calculated by taking into account only the area above a noise level N which in many cases can be neglected if the signal to noise ratio (SNR) is large enough. The quotients based on the FFT calculations can be based on the amplitude from the echoes or the intensities from the echoes which is the square of the amplitude. The temperature of the target area relating to the measurements made is, according to the invention, not calculated on the basis of one of said quotients or a combination of said quotients.

In experiments the inventor has based the calculations on all types of quotient using all harmonics in different combinations to find the most sensitive combination of one or many quotients. The above given examples are the most frequently used quotients. However this is tissue dependent and must be experimentally investigated from cases to cases. Depending on the result the non-invasive treatment is repeated under temperature control according to the procedure described until the desired temperature in the target area T (tissue or inserted artificial material) or a shell around the target area has been developed.

Other non-linear calculation systems can be proposed in order to increase the precision of the calculation of the temperature change such as

$$\Delta T = \text{function (quotient)}$$

wherein the function has to be determined from case to case.

In heating a stent covered by an ultrasound absorbing and reflecting material such as polytetrafluoro ethylene, polyurethane or elastomer it should be possible to measure in the range from 37 to about 55°c and in connection with treatment of cancer up to 85°C. For therapeutical treatment of e.g. muscles it is desired to measure up to 41°C in order to avoid a higher temperature.

Preferred embodiments have been described in order to illustrate the invention but it is obvious to the man skilled in the art that these embodiments are examples only and that modifications thereof can be made without departing from the scope of the invention as defined in the claims.

**Claims**

1. Method of non-invasive measuring of the temperature change of a target inside a body by transmitting an ultrasonic pulse to the target, subjecting a pulse reflected from the target to frequency analysis, and calculating the temperature change of the target there from, wherein a frequency spectrum of the reflected pulse is produced, **characterized in that** the temperature change is calculated to be proportional to the quotient of the amplitude of harmonics of said spectrum.

2. The method of claim 1 wherein the calculation includes calculation of a quotient

$$\frac{Akn - Ak0}{Ak0}$$

wherein Ak0 represents the size of a frequency peak of a harmonic of number k, based on amplitude or intensity as measured at an earlier moment and Akn represents the size of a frequency peak of said harmonic as measured at a later moment n.

3. The method of claim 2 wherein the temperature change is calculated according to the relationship

$$\Delta T = k \cdot Q$$

wherein k is a constant determined empirically, and Q is the quotient of claim 2.

4. The method of claim 2 wherein the quotient is calculated for different harmonics represented by frequency peaks in said spectrum.

5. The method of claim 2 wherein the size of the harmonic is represented by the amplitude of the frequency peak representing the harmonic in the frequency spectrum.

6. The method of claim 2 wherein the size of the harmonic is represented by the surface defined by the frequency peak representing the harmonic in the frequency spectrum.

7. The method of claim 7 wherein the surface extends from a noise level defined by the frequency spectrum.

8. Apparatus for non-invasive measuring of the temperature change of a target inside a body comprising means for transmitting an ultrasonic pulse to the target, means for receiving a pulse reflected from the target means for frequency analysis of the reflected pulse, means for calculating the temperature change of the target therefrom, means for producing a frequency spectrum of the reflected pulse, and means for calculation of the temperature change on the basis of harmonics appearing in said spectrum, **characterized in that** said means for calculation of the temperature change calculates the temperature change as being proportional to the quotient of the amplitude of harmonics of said spectrum.

**Patentansprüche**

1. Verfahren zur nichtinvasiven Messung der Temperaturveränderung eines Zieles innerhalb eines Körpers durch die Übertragung eines Ultraschallpulses auf das Ziel, die Durchführung einer Frequenzanalyse des vom Ziel reflektierten Pulses und die Berechnung der Temperaturveränderung des Zieles aus dieser, wobei ein Frequenzspektrum des reflektierten Pulses erzeugt wird, **dadurch gekennzeichnet, daß** die Temperaturveränderung als proportional zum Quotienten der Amplitude der Oberschwingungen des besagten Spektrums berechnet wird.

2. Das Verfahren gemäß Anspruch 1, wobei die Berechnung die Ermittlung eines Quotienten

$$\frac{Akn - Ak0}{Ak0}$$

umfaßt, wobei Ak0 die Größe einer Frequenzspitze einer Oberschwingung der Zahl k, basierend auf der Amplitude oder Intensität, gemessen zu einem früheren Zeitpunkt, repräsentiert und Akn die Größe einer Frequenzspitze der besagten Oberschwingung gemessen zu einem späteren Zeitpunkt n repräsentiert.

3. Das Verfahren gemäß Anspruch 2, wobei die Temperaturveränderung gemäß der Beziehung

$$\Delta T = k \cdot Q$$

berechnet wird, wobei k eine empirisch ermittelte Konstante und Q der Quotient gemäß Anspruch 2 ist.

4. Das Verfahren gemäß Anspruch 2, wobei der Quotient für verschiedene Oberschwingungen, repräsentiert durch Frequenzspitzen im besagten Spektrum, berechnet wird.

5. Das Verfahren gemäß Anspruch 2, wobei die Größe der Oberschwingung durch die Amplitude der Frequenzspitze repräsentiert wird, die die Oberschwingung im Frequenzspektrum repräsentiert.

6. Das Verfahren gemäß Anspruch 2, wobei die Größe der Oberschwingung durch die Oberfläche der Frequenzspitze repräsentiert wird, die die Oberschwingung im Frequenzspektrum repräsentiert.

7. Das Verfahren gemäß Anspruch 2, wobei sich die Oberfläche von einem durch das Frequenzspektrum definierten Rauschpegel aus erstreckt.

8. Gerät für die nichtinvasive Messung der Temperaturveränderung eines Zieles in einem Körper, bestehend aus Mitteln für die Übertragung eines Ultraschallpulses auf das Ziel, Mitteln für den Empfang eines vom Ziel reflektierten Pulses, Mitteln für die Frequenzanalyse des reflektierten Pulses, Mitteln für die Berechnung der Temperaturveränderung des Zieles aus dieser, Mitteln für die Erzeugung eines Frequenzspektrums des reflektierten Pulses und Mitteln für die Berechnung der Temperaturveränderung auf der Grundlage von Oberschwingungen in dem besagten Spektrum, **dadurch gekennzeichnet, daß** die besagten Mittel für die Berechnung der Temperaturveränderung die Temperaturveränderung als proportional zum Quotienten aus der Amplitude der Oberschwingungen des besagten Spektrums berechnen.

**Revendications**

1. Procédé de mesure non invasive du changement de température d'une cible à l'intérieur d'un corps par la transmission d'une impulsion ultrasonore à la cible, la soumission d'une impulsion réfléchie de la cible à une analyse de fréquence, et le calcul du changement de température de la cible à partir de celle-ci, dans lequel un spectre de fréquence de l'impulsion réfléchie est produit, **caractérisé en ce que** le changement de température est calculé de façon à être proportionnel au quotient de l'amplitude des harmoniques dudit spectre.

2. Procédé selon la revendication 1, dans lequel le calcul comprend le calcul d'un quotient

$$\frac{Akn - Ak0}{Ak0}$$

dans lequel Ak0 représente la taille d'un pic de fréquence d'un harmonique de nombre k, sur la base d'une amplitude ou d'une intensité mesurée à un moment antérieur et Akn représente la taille d'un pic de fréquence dudit harmonique mesurée à un moment ultérieur n.

3. Procédé selon la revendication 2, dans lequel le changement de température est calculé selon la relation

$$\Delta T = k * Q$$

k étant une constante déterminée empiriquement et Q étant le quotient selon la revendication 2.

4. Procédé selon la revendication 2, dans lequel le quotient est calculé pour différents harmoniques représentés par des pics de fréquence dans ledit spectre.

5. Procédé selon la revendication 2, dans lequel la taille de l'harmonique est représentée par l'amplitude du pic de fréquence représentant l'harmonique dans le spectre de fréquence.

6. Procédé selon la revendication 2, dans lequel la taille de l'harmonique est représentée par la surface définie par le pic de fréquence représentant l'harmonique dans le spectre de fréquence.

7. Procédé selon la revendication 7, dans lequel la surface s'étend depuis un niveau de bruit défini par le spectre de fréquence.

8. Appareil de mesure non invasive du changement de température d'une cible à l'intérieur d'un corps, comprenant un moyen de transmission d'une impulsion ultrasonore à la cible, un moyen permettant de recevoir une impulsion réfléchie à partir du moyen de cible pour l'analyse de fréquence de l'impulsion réfléchie, un moyen de calcul du changement de température de la cible à partir de celle-ci, un moyen de production d'un spectre de séquence de l'impulsion réfléchie et un moyen de calcul du changement de température sur la base des harmoniques apparaissant dans ledit spectre, **caractérisé en ce que** ledit moyen de calcul du changement de température calcule le changement de température comme étant proportionnel au quotient de l'amplitude des harmoniques dudit spectre.

FIG. 1

FIG. 2

Frequency range

First harmonic

Second harmonic

Third harmonic

1E-3
1E-4
1E-5
1E-6
1E-7
1E-8
1E-9
1E-10

0,000   1,000M   2,000M   3,000M   4,000M   5,000M   6,000M

## FIG. 3

Amplitude

Quotients

$-Y1 = f_0$; time = start time 0;

time = measuring 1 after start

$\text{Quotient} = \dfrac{Y11 - Y10}{Y10}$

Y 10

Y 20

N

Y 30

$f_0$        $2f_0$        $3f_0$

## FIG. 4

Amplitude

Quotients

$-Y1 = f_0$; time = start time 0;

time = measuring 1 after start

$\text{Quotient} = \dfrac{Y11 - Y10}{Y10}$

Y 10

Y 20

N

Y 30

$f_0$        $2f_0$        $3f_0$

## FIG. 5

**EP 1 562 480 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1233337 A
- US 5370121 A
- US 4807633 A
- US 6050943 A
- US 4566460 A
- US 5360268 A
- US 4817615 A
- US 4754760 A
- JP 61280533 B

**Non-patent literature cited in the description**

- **SEIP R. et al.** Invasive and Non-invasive Feedback for Ultrasound Phased Array Thermometry. *IEEE Ultrasonics Symposium,* 1994, 1821-1824